# EUROPEAN PATENT APPLICATION

(11) **EP 2 168 565 A1**
(43) Date of publication of application: **31.03.2010**
(21) Application number: 08777793.4
(22) Date of filing: 02.07.2008
(51) Int. Cl.: A61K 8/29, A61K 8/26, A61K 8/89, A61Q 1/02

(54) **COMPOSITION FOR CONCEALING DAMAGE SCAR ON SKIN**

(30) Priority: 04.07.2007 JP 2007175858
(71) Applicant: Shiseido Company, Ltd., Chuo-ku Tokyo 104-8010 (JP)
(72) Inventor: MASACHIKA, Kiriko, Yokohama-shi Kanagawa 224-8558 (JP)
(74) Representative: Lippert, Stachow & Partner
(86) International application number: PCT/JP2008/061993
(87) International publication number: WO 2009/005090

(57) **Abstract**

The present invention provides a composition for correcting scar of skin which corrects a scar of skin such as a mark of skin injury, which achieves an excellent effect of correcting a scar of skin with a good covering ability and an ease in applying the composition repeatedly or another thereover and has a good feeling in use as well as an excellent long-lasting effect in makeup. The composition comprises (a) 30 to 50 % by mass of titanium oxide and (b) 2 to 20 % by mass of coating agents including acrylic-silicone graft copolymer and siliconated polysaccharide compound.

## Description

### RELATED APPLICATIONS

This application claims the priority of Japanese Patent Application No. 2007-175858 filed on July 4, 2007, which is incorporated herein by reference.

### FIELD OF THE INVENTION

The present invention relates to a composition for correcting scar of skin, and in particular, relates to a composition for correcting scar of skin which corrects a scar of skin such as a mark of skin injury.

### BACKGROUND OF THE INVENTION

Conventionally, to conceal a scar of skin such as a mark of skin injury, covering the scar with thick application of cosmetics has been applied as the way to make the scar less noticeable.
Depending on degree of scar, however, there are many cases that a generally-used cosmetic could not correct sufficiently a scar of skin such as a mark of skin injury. Thus, the cosmetic which is specially used for a portion of a scar of skin has been demanded.
On the other hand, a titanium oxide, which is the powder used in the present invention, is generally used as powder for cosmetics. Examples of the formulation with a large amount of titanium oxide include Example 3 in Patent Literature 1 and Examples 1 and 2 in Patent Literature 2. However, they did not achieve a sufficient effect of correcting scars, and they provided tautness in the finished makeup.
Moreover, examples of the cosmetic containing a coating agent include Patent Literatures 3 and 4. However, such cosmetics have a difficulty in applying the cosmetic repeatedly or another thereover and disadvantages such as insufficient effects of correcting scars and poor long-lasting effects in makeup.

Patent Literature 1: Japanese Unexamined Patent Publication H08-81331
Patent Literature 2: Japanese Unexamined Patent Publication H07-89826
Patent Literature 3: Japanese Unexamined Patent Publication 2000-38313
Patent Literature 4: Japanese Unexamined Patent Publication 2002-138009

### DISCLOSURE OF THE INVENTION

### PROBLEM TO BE SOLVED BY THE INVENTION

The present invention was made in view of the above-described conventional problems. Thus, the object of the present invention is to provide a composition for correcting scar of skin which achieves an excellent effect of correcting a scar of skin with a good covering ability and an ease in applying the composition repeatedly or another thereover and has a good feeling in use as well as an excellent long-lasting effect in makeup.

### MEANS TO SOLVE THE PROBLEM

The present inventors have studied variously to obtain a composition for correcting scar of skin which achieves an excellent effect of correcting scar of skin and has an excellent feeling in use. As a result, the inventors have found that the above-mentioned problem could be solved by using a specific powder and specific coating agents, thus leading to completion of the present invention.

The present invention provides a composition for correcting scar of skin comprising: (a)30 to 50 % by mass of titanium oxide and (b)2 to 20 % by mass of coating agents including an acrylic-silicone graft copolymer and a siliconated polysaccharide compound.

### EFFECT OF THE INVENTION

The composition for correcting scar of skin of the present invention achieves an excellent effect of correcting scar of skin with a good covering ability and an ease in applying the composition repeatedly or another thereover and has a good feeling in use as well as an excellent long-lasting effect in makeup.

### BEST MODE FOR CARRYING OUT THE INVENTION

Hereinafter, the best mode for carrying out the present invention is described.
The titanium oxide used as Component (a) in the present invention is a pigment class titanium oxide having an average particle diameter of 0.3 µm or more. The blending amount of the titanium oxide is 30 to 50 % by mass, more preferably 35 to 45 % by mass. When it is less than 30 % by mass, the covering ability is poor. When it exceeds 50 % by mass, the applied skin looks like having too much makeup, resulting in unnatural finish.

The coating agents used as Component (b) in the present invention contain an acrylic-silicone graft copolymer and a siliconated polysaccharide compound. In the following, these coating agents will be described in detail.

An acrylic-silicone graft copolymer is a copolymer which is formed from an organopolysiloxane compound having a radical polymerizable group at the one terminal of its molecular chains and a radical polymerizable monomer mainly comprising acrylate and/or methacrylate. Examples of such acrylic-silicone graft copolymer include the ones described in Japanese Unexamined Patent Publications H02-25411, H02-132141, and so on. Examples of the organopolysiloxane compound having a radical polymerizable group at the one terminal of its molecular chains include the one represented by the following formula (1).

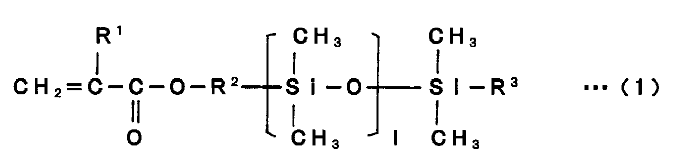

In the formula (1), R¹ represents a methyl group or a hydrogen atom, R² represents a divalent saturated hydrocarbon group having 1 to 10 carbon atoms with linear or branched carbon chains wherein one or two ether bonds may be present within the carbon chains, R³ represents a methyl group or a butyl group, and 1 represents 3 to 300.

A radical polymerizable monomer mainly comprising acrylate and/or methacrylate is a compound having one radical polymerizable unsaturated bond in its molecule. Examples of the acrylate and/or methacrylate used include alkyl(metha)acrylate such as methyl(metha)acrylate, ethyl(metha)acrylate, n-butyl(metha)acrylate, 2-ethylhexyl(metha)acrylate, stearyl(metha)acrylate, and behenyl(metha)acrylate; hydroxyalkyl(metha)acrylate such as 2-hydroxyethyl(metha)acrylate and 2-hydroxypropyl(metha)acrylate; and perfluoroalkyl(metha)acrylate having 1 to 10 fluoro-carbon chains. For the radical polymerizable monomer mainly comprising acrylate and/or methacrylate in the present invention, various polymerizable monomer compounds can be used in addition to the above-mentioned acrylates and/or methacrylates as necessary. Examples of such compounds include styrene, substituted styrene, vinyl acetate, (metha)acrylic acid, maleic anhydride, maleate, fumarate, vinyl chloride, vinylidene chloride, ethylene, propylene, butadiene, acrylonitrile, olefin fluoride, and N-vinylpyrrolidone.

A copolymerization of (A) a dimethylpolysiloxane compound having a radical polymerizable group at the one terminal of its molecular chains and (B) a radical polymerizable monomer mainly comprising acrylate and/or methacrylate is carried out within the copolymerization ratio range of ((A)/(B)):1/19 to 2/1, in the presence of a common radical polymerization initiator such as benzoyl peroxide, lauroyl peroxide, and azobisisobutyronitrile. Any of solution polymerization, emulsion polymerization, suspension polymerization, and bulk polymerization can be applied in the present invention. Examples of commercially-available copolymer, which is dissolved in a solvent, include KP541, KP543, and KP545 (all of them are manufactured by Shin-Etsu Chemical Co., Ltd.)
One or more of these acrylic-silicone graft copolymers can be used as necessary.

The siliconated polysaccharide compound used in the present invention is represented by the following formula (2).

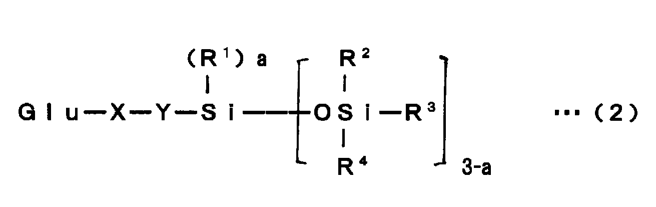

In the formula (2), Glu represents a sugar residue of the polysaccharide compound, X represents a divalent bonding group, Y represents a divalent aliphatic group, R¹ represents a monovalent organic group having 1 to 8 carbon atoms, each of R², R³, and R⁴ represents a monovalent organic group having 1 to 8 carbon atoms or a siloxy group represented by -OSiR⁵R⁶R⁷, each of R⁵, R⁶, and R⁷ represents a monovalent organic group having 1 to 8 carbon atoms, and a represents 0, 1, or 2.

In the formula (2), Glu represents a sugar residue of the polysaccharide compound, and examples of such polysaccharide compound include various kinds of known polysaccharide compound such as cellulose, hemicellulose, gum arabic, tragacanth gum, tamarind gum, pectin, starch, mannan, guar gum, locust bean gum, quince seed gum, alginic acid, carrageenan, agar, xanthan gum, dextran, pullulan, chitin, chitosan, hyaluronic acid, and chondroitin sulfate as well as the derivatives of these polysaccharide compounds, such as carboxymethylated derivatives, sulfated derivatives, phosphorylated derivatives, methylated derivatives, ethylated derivatives, derivatives obtained through addition of alkylene oxide such as ethylene oxide and propylene oxide, acylated derivatives, cationized derivatives, and low molecular weight derivatives. Among these polysaccharide compounds, ethylcellulose or pullulan is preferably used, and pullulan is particularly preferred. In the present invention, the average molecular weight of the polysaccharide compound varies depending on its kind, and generally about 1,000 to 5,000,000 is preferred.

Each of these polysaccharide compounds contains at least one of one or more reactive functional groups such as a hydroxyl group and a carboxyl group depending on its kind. The divalent bonding group represented by X is a bonding group derived from A which is formed through reaction between a reactive functional group of the polysaccharide compound and a silicone compound represented by the following formula (3). For such reaction between the silicone compound and the polysaccharide compound, a conventionally known method can be used.

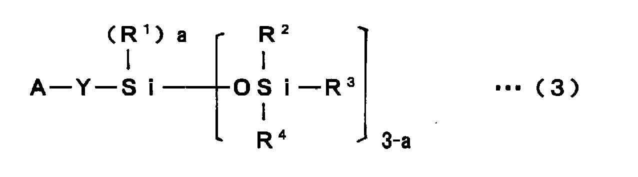

In the formula (3), each of Y, R¹, R², R³, R⁴, and a represents the same in the above-mentioned formula (2). A is a functional group which can react with the reactive functional group of the polysaccharide compound. Examples of such functional group include an isocyanate group, an epoxy group, a vinyl group, an acryloyl group, a methacryloyl group, an amino group, an imino group, a hydroxyl group, a carboxyl group, and a mercapto group.

Examples of X include a carbamoyl group, -CH₂CH(OH)-, a carbonyl group, an amino group, and an ether group. In terms of reactivity, a carbamoyl group (-CONH-), which is formed through reaction between the compound represented by the above-mentioned formula (3) in which A is an isocyanate group (O=C=N-) and a hydroxyl group of the polysaccharide compound, is preferable. In this case, the sugar residue of the polysaccharide compound refers to a residual portion derived by removing a hydrogen atom of the hydroxyl group reacted with the isocyanate group from the polysaccharide compound. In a case that any other kind of reactions is carried out, the sugar residue of the polysaccharide compound is to be understood to mean likewise.

Examples of the divalent aliphatic group represented by Y include an alkylene group, an alkylene group having an oxygen atom, a nitrogen atom, and/or a sulfur atom in its main chains, an alkylene group having an arylene group such as a phenylene group in its main chains, and an alkylene group having a carbonyloxy group or an oxycarbonyl group in its main chains. Each of these divalent aliphatic groups can have a substituent group such as a hydroxy group, an alkoxy group, and/or an alkyl group, and a terminal atom of the aliphatic group can be a hetero atom such as an oxygen atom, a nitrogen atom, or a sulfur atom. Examples of Y include -(CH₂)₂-, -(CH₂)₃-, -(CH₂)₄-, -(CH₂)₆-, -(CH₂)₈-, -[CH₂CH(CH₃)]-, -(CH₂)₂O(CH₂)₃-, and -CH₂CH(OH)-CH₂-, and preferably a propylene group represented by -(CH₂)₃-.

In the above-mentioned formula (2), examples of the monovalent organic group having 1 to 8 carbon atoms, which is represented by R¹, R², R³, R⁴, R⁵, R⁶, and R⁷, include alkyl groups such as a methyl group, an ethyl group, a propyl group, and a butyl group; cycloalkyl groups such as a cyclopentyl group and a cyclohexyl group; aryl groups such as a phenyl group; aralkyl groups such as a benzyl group; alkenyl groups such as a vinyl group and an allyl group; and fluorinated alkyl groups such as a 3,3,3-trifluoropropyl group.

Each of R², R³, and R⁴ can be a siloxy group represented by -oSiR⁵R⁶R⁷. Examples of such siloxy group include a trimethylsiloxy group, an ethyldimethylsiloxy group, a phenyldimethylsiloxy group, a vinyldimethylsiloxy group, and a 3,3,3-trifluoropropyl dimethylsiloxy group. R¹, R², R³, R⁴, R⁵, R⁶, and R⁷ can be same with or different from one another. In the siliconated polysaccharide compound of the present invention, it is particularly preferred that a = 0 and each of R², R³, and R⁴ represents a methyl group.

The particularly preferable siliconated polysaccharide compound used in the present invention is a siliconated pullulan represented by the following formula (4). In the formula (4), PL represents a glucose residue of the pullulan.

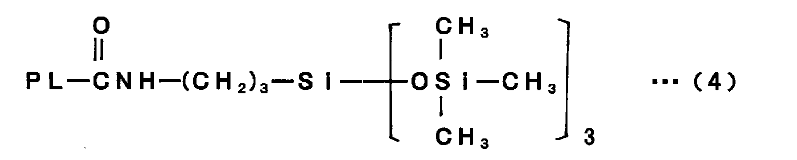

In the siliconated polysaccharide compound of the present invention, the bonding ratio of the silicone compound to the reactive functional group of the polysaccharide compound varies depending on the kind. It is generally preferred that the average bonding number (substitution degree) of the silicone compound per one sugar unit constituting the polysaccharide compound is 0.5 to 3.0. In the present invention, the substitution degree is calculated on the basis of the amount (wt. %) of Si in the siliconated polysaccharide compound.
An ease of blending and a feeling in use can be improved by dissolving the siliconated polysaccharide compound in a low-molecular-weight silicone oil or a light isoparaffin before blending.

One or more of these siliconated polysaccharide compounds can be used as necessary. Examples of commercially-available siliconated polysaccharide compound include TSPL=30-D5 (manufactured by Shin-Etsu Chemical Co., Ltd.: 30 wt% in decamethylcyclopentasiloxane solution).

In the composition for correcting scar of skin of the present invention, the amount of the coating agents including the acrylic-silicone graft copolymer and the siliconated polysaccharide compound can be 2 to 20 % by mass with respect to the composition, more preferably 2 to 15 % by mass. When the amount of the coating agents is too small, the composition comes to have a difficulty in applying repeatedly or another thereover and has a poor long-lasting effect in makeup. When the amount of the coating agents is too much, the spreadability becomes deteriorated or tautness is felt in finished makeup.

It is preferred that the coating agents used consist of the acrylic-silicone graft copolymer and the siliconated polysaccharide compound. However, other coating agents can be blended in addition to the above-mentioned agents as long as the blending amount of the other coating agents is 10 % by mass or less with respect to the total amount of the coating agents.
Examples of such coating agents include a polyvinylpyrrolidone/alpha-olefin copolymer, a trimethyl siloxysilicate polymer, and an amino-modified silicone. When a large amount of these coating agents is blended, the composition comes to have disadvantages such as lacking an ease in applying repeatedly or another thereover and a long-lasting effect in makeup and providing tautness in the finished makeup.

It is preferred that the blending ratio (mass ratio) of the acrylic-silicone graft copolymer : the siliconated polysaccharide compound is 1:0.1 to 1:0.3.
When the blending ratio of the acrylic-silicone graft copolymer is too high, the composition comes to lack flexibility, resulting in a difficulty in applying repeatedly or another thereover and a poor long-lasting effect in makeup. When the blending ratio of the siliconated polysaccharide compound is too high, the composition may provide stickiness, a heavy feeling in use, or a poor affinity to skin.

In the present invention, it is preferred that a calcined mica is further blended in addition to the above-mentioned components. A better spreadability and an improved usability can be achieved by blending a calcined mica. The desired amount of the calcined mica is 40 % by mass or less with respect to the total amount of the composition for correcting scar of skin, preferably 5 to 20 % by mass.

In the present invention, other powders can be blended, for example inorganic powders such as talc, kaolin, mica, sericite, muscovite, biotite, phlogopite, synthetic mica, vermiculite, magnesium carbonate, calcium carbonate, aluminum silicate, barium silicate, calcium silicate, magnesium silicate, metal tungstate, magnesium, silica, zeolite, barium sulfate, calcined calcium sulfate, calcined gypsum, calcium phosphate, fluorine apatite, hydroxyapatite, ceramic powder, and metallic soap (e.g., zinc myristate, calcium palmitate, and aluminum stearate); organic powders such as polyamide resin powder, polyethylene powder, polymethyl methacrylate powder, polystyrene powder, styrene-acrylic acid copolymer resin powder, benzoguanamine resin powder, polytetrafluoroethylene powder, and cellulose powder; inorganic white pigments such as titanium dioxide and zinc oxide; inorganic red pigments such as iron oxide (red iron oxide) and iron titanate; inorganic brown pigments such as gamma-iron oxide; inorganic yellow pigments such as yellow iron oxide and loess; inorganic black pigments such as black iron oxide and lower titanium oxide; inorganic purple pigments such as mangoviolet and cobaltviolet; inorganic green pigments such as chromium oxide, chromium hydroxide, and cobalt titanate; inorganic blue pigments such as ultramarine and Prussian blue; pearl pigments such as titanium oxide-coated mica, titanium oxide-coated bismuth oxychloride, titanium oxide-coated talc, colored-titanium oxide-coated mica, bismuth oxychloride, and fish scale foil; metal powder pigments such as aluminum powder and copper powder; organic pigments such as Red No.202, Red No.205, Red No.220, Red No.228, Red No.405, Orange No.203, Orange No.204, Yellow No.205, Yellow No.401, and Blue No.404; organic pigments such as zirconium, barium, and aluminum lake (e.g., Red No.3, Red No.104, Red No.227, Red No.401, Orange No.205, Yellow No.4, Yellow No. 202, Green No.3, and Blue No.1); and natural colorants such as chlorophyll and β-carotene.

Additionally, in the composition for correcting scar of skin of the present invention, other components generally used in cosmetics can be blended so far as the effect of the present invention is not deteriorated. For such other components which can blended in the composition of the present invention, examples of the aqueous phase component include moisturizers such as polyhydric alcohol, mucopolysaccharides (e.g., sodium hyaluronnate), organic acid and organic salts (e.g., amino acid, amino acid salt, and oxy acid salt); example of the oil phase components include solid/semi-solid oils (e.g., petrolatum, lanolin, silicone wax, higher fatty acid, and higher alcohol), liquid oils (e.g., squalane, liquid paraffin, ester oil, triglyceride, volatile hydrocarbon oil, and fluorocarbon), surfactants (e.g., cationic surfactant, anionic surfactant, and nonionic surfactant), drugs (e.g., vitamin E and vitamin E acetate), pH adjusters (e.g., disodium phosphate), astringent agent, antioxidant, preservative, perfume, clay mineral, thickener, and UV absorber.

The composition for correcting scar of skin of the present invention is preferably used as a foundation, a pre-makeup, or a concealer (partial-coverage pre-makeup).

### EXAMPLES

Hereinafter, the examples of the present invention are explained. The present invention is not limited by these examples. All of the amounts are expressed in mass %.

### Test Examples 1 to 13

### (Formulation)

Compositions for correcting scar of skin were prepared according to the formulations showed in the following Table 1, and effects of each composition were evaluated with an evaluation method described below. The results were showed in Tables 1 and 2.

**Table 1**

| Test Examples | | 1 | 2 | 3 | 4 | 5 | 6 | 7 | 8 |
|---|---|---|---|---|---|---|---|---|---|
| Titanium oxide | Titanium oxide | 30 | 30 | 25 | 35 | 30 | 30 | 30 | 30 |
| Plate-like powder | Calcined mica | 15 | 20 | 20 | 3 | 20 | 20 | 20 | 20 |
| | Talc | - | - | - | 10 | - | - | - | - |
| Coating agent | Acrylic-silicone graft copolymer | 1 | 0.5 | 1 | 1 | - | 5 | 5 | 5 |
| | Siliconated polysaccharide compound (30 wt% in decamethylcyclopentasiloxane solution) | 1 | 0.5 | 1 | 1 | - | - | - | - |
| | Polyvinylpyrrolidone/alpha-olefin copolymer | - | - | - | - | - | - | 5 | - |
| | Trimethyl siloxysilicate polymer | - | - | - | - | - | - | - | 5 |
| Volatile oil | Decamethylcyclopentasiloxane | 15 | 15 | 20 | 20 | 15 | 15 | 10 | 10 |
| Wax | Ceresin | 2 | 2 | 2 | 2 | 2 | 2 | 2 | 2 |
| | Polyethylene wax (average molecular weight 500) | 4 | 4 | 4 | 4 | 4 | 4 | 4 | 4 |
| Oil | Squalane | 14 | 15 | 14 | 11 | 16 | 11 | 11 | 11 |
| | Dimethylpolysiloxane | 10 | 5 | 5 | 5 | 5 | 5 | 5 | 5 |
| Powder | Iron-oxide pigment | 8 | 8 | 8 | 8 | 8 | 8 | 8 | 8 |
| | Total | 100 | 100 | 100 | 100 | 100 | 100 | 100 | 100 |
| Covering ability | | ⊚ | ⊚ | △ | ⊚ | ⊚ | ⊚ | ⊚ | ⊚ |
| Spreadability | | ○ | ○ | ○ | ○ | ○ | ○ | ○ | ○ |
| Non heavy-makeup appearance | | ○ | ○ | ○ | ○ | ○ | ○ | ○ | ○ |
| Moderately-matte finish | | ○ | ○ | △ | ○ | ○ | ○ | ○ | ○ |
| No-tautness in finish | | ○ | ○ | ○ | ○ | ○ | ○ | ○ | X |
| Ease in applying the composition repeatedly | | ⊚ | △ | ⊚ | ⊚ | △ | △ | △ | △ |
| Long-lasting effect in makeup (2 hours) | | ⊚ | ○ | ○ | ⊚ | △ | △ | ○ | ○ |

**Table 2**

| Test Examples | | 9 | 10 | 11 | 12 | 13 |
|---|---|---|---|---|---|---|
| Titanium oxide | Titanium oxide | 50 | 55 | 30 | 30 | 30 |
| Plate-like powder | Calcined mica | 3 | 3 | 15 | 15 | 20 |
| | Talc | - | - | - | - | - |
| Coating agent | Acrylic-silicone graft copolymer | 1 | 1 | 15 | 20 | - |
| | Siliconated polysaccharide compound(30 wt% in decamethylcyclopentasiloxane solution) | 1 | 1 | 5 | 5 | 5 |
| | Polyvinylpyrrolidone/alpha-olefin copolymer | - | - | - | - | - |
| | Trimethyl siloxysilicate polymer | - | - | - | - | - |
| Volatile oil | Decamethylcyclopentasiloxane | 15 | 15 | 15 | 15 | 15 |
| Wax | Ceresin | 2 | 2 | 2 | 2 | 2 |
| | Polyethylene wax (average molecular weight 500) | 4 | 4 | 4 | 4 | 4 |
| Oil | Squalane | 6 | 5 | 3 | 0.5 | 11 |
| | Dimethylpolysiloxane | 10 | 6 | 3 | 0.5 | 5 |
| Powder | Iron-oxide pigment | 8 | 8 | 8 | 8 | 8 |
| | Total | 100 | 100 | 100 | 100 | 100 |
| Covering ability | | ⊚ | ⊚ | △ | ⊚ | ⊚ |
| Spreadability | | ○ | ○ | ○ | △ | ○ |
| Non heavy-makeup appearance | | ○ | X | ○ | ○ | ○ |
| Moderately-matte finish | | ○ | ○ | ○ | ○ | ○ |
| No-tautness in finish | | ○ | ○ | ○ | X | ○ |
| Ease in applying the composition repeatedly | | ⊚ | △ | ⊚ | ⊚ | △ |
| Long-lasting effect in makeup (2 hours) | | ⊚ | ○ | ○ | ⊚ | △ |

### (Method for preparing compositions for the correcting scar of skin in Tables 1 and 2)

The components were dissolved by heating at 90 to 100 °C, and then stirred and mixed, defoamed, and cooled.

### [Evaluation method]

6 professional panelists used each of the compositions for correcting scar of skin prepared in the above-mentioned Test Examples 1 to 13 and sensory-evaluated each of them in five levels in terms of covering ability, spreadability, non heavy-makeup appearance, moderately-matte finish, no-tautness in finish, ease in applying the composition repeatedly, and long-lasting effect in makeup after 2 hours. The evaluation criteria are as follows.

### [Evaluation criteria]

5: Very good
4: Good
3: Average
2: Poor
1: Very poor

### [Evaluation]

⊚: Evaluation value (average) is 4.5 or more
○: Evaluation value (average) is 3.5 or more and less than 4.5
□: Evaluation value (average) is 2.5 or more and less than 3.5
△: Evaluation value (average) is 1.5 or more and less than 2.5
X: Evaluation value (average) is less than 1.5

In Tables 1 and 2, Test Examples 1, 4, 9, and 11 are the compositions according to the example of the present invention, and Test Examples 2, 3, 5 to 8, 10, 12, and 13 are the comparative examples. As is clear from Tables 1 and 2, the compositions for correcting scar of skin of the present invention is excellent in covering ability, spreadability, non heavy-makeup appearance, moderately-matte finish, no-tautness in finish, ease in applying the composition repeatedly, and long-lasting effect in makeup.

## Claims

1. A composition for correcting scar of skin comprising:
(a)30 to 50 % by mass of titanium oxide and
(b) 2 to 20 % by mass of coating agents including an acrylic-silicone graft copolymer and a siliconated polysaccharide compound.

2. The composition for correcting scar of skin according to Claim 1, further comprising a calcined mica.

3. The composition for correcting scar of skin according to Claim 1, wherein the acrylic-silicone graft copolymer and the siliconated polysaccharide compound as the component (b) are blended in a ratio (mass ratio) of acrylic-silicone graft copolymer : siliconated polysaccharide compound = 1:0.1 to 1:0.3.

4. The composition for correcting scar of skin according to Claim 1, wherein the acrylic-silicone graft copolymer in the component (b) is a copolymer of an organopolysiloxane compound having a radical polymerizable group at the one terminal of its molecular chains represented the following formula (1) and a radical polymerizable monomer mainly comprising acrylate and/or methacrylate: 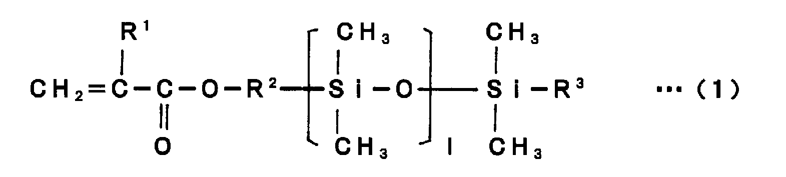 wherein R¹ represents a methyl group or a hydrogen atom, R² represents a divalent saturated hydrocarbon group having 1 to 10 carbon atoms with linear or branched carbon chains wherein one or two ether bonds may be present within the carbon chains, R³ represents a methyl group or a butyl group, and 1 represents 3 to 300.

5. The composition for correcting scar of skin according to Claim 1, wherein the siliconated polysaccharide compound in the component (b) is represented by the following formula (2): 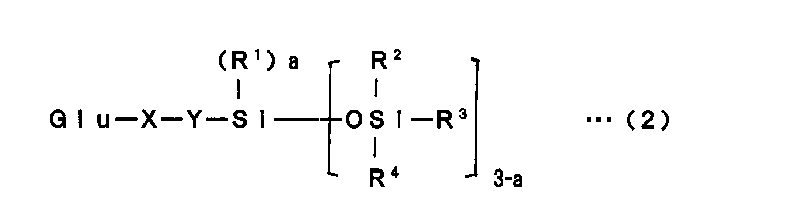 wherein Glu represents a sugar residue of the polysaccharide compound, X represents a divalent bonding group, Y represents a divalent aliphatic group, R¹ represents a monovalent organic group having 1 to 8 carbon atoms, each of R², R³, and R⁴ represents a monovalent organic group having 1 to 8 carbon atoms or a siloxy group represented by -OSiR⁵R⁶R⁷, each of R⁵, R⁶, and R⁷ represents a monovalent organic group having 1 to 8 carbon atoms, and a represents 0, 1, or 2.
